# EUROPÄISCHE PATENTSCHRIFT

(11) **EP 0 994 836 B1**
(45) Veröffentlichungstag und Bekanntmachung des Hinweises auf die Patenterteilung: **02.10.2002**
(21) Anmeldenummer: 98939524.9
(22) Anmeldetag: 22.06.1998
(51) Int. Cl.: C07C 37/84, C07C 37/14, C07C 37/52, C07C 39/17

(54) **HERSTELLUNG UND REINIGUNG VON 3-(4-HYDROXYPHENYL)-1,1,3-TRIMETHYLINDAN-5-OL**
METHOD FOR PRODUCING AND PURIFYING 3-(4-HYDROXYPHENYL)-1,1,3-TRIMETHYLINDAN-5-OL
PROCEDE DE PREPARATION ET D'EPURATION DE 3-(4-HYDROXYPHENYL)-1,1,3-TRIMETHYLINDAN-5-OL

(30) Priorität: 03.07.1997 DE 19728377
(43) Veröffentlichungstag der Anmeldung: 26.04.2000
(73) Patentinhaber: BAYER AG, 51368 Leverkusen (DE)
(72) Erfinder: WESTERNACHER, Stefan, Seabrook, TX 77586 (US); KOSIK, Franz, D-47647 Kerken (DE); CALAMINUS, Wolfgang, D-47800 Krefeld (DE); HAESE, Wilfried, D-51519 Odenthal (DE)
(86) Internationale Anmeldenummer: EP9803801
(87) Internationale Veröffentlichungsnummer: WO99001415

(56) Entgegenhaltungen:
- DE-A- 2 645 020
- US-A- 3 288 864
- US-A- 4 334 106
- DATABASE WPI Section Ch, Week 7532 Derwent Publications Ltd., London, GB; Class E14, AN 75-53040W XP002081238 & JP 50 035150 A (MITSUI TOATSU CHEM INC) , 3. April 1975 in der Anmeldung erwähnt
- DATABASE WPI Section Ch, Week 7930 Derwent Publications Ltd., London, GB; Class A60, AN 79-55597B XP002081239 & JP 54 076564 A (MITSUI TOATSU CHEM INC) , 19. Juni 1979
- DATABASE WPI Section Ch, Week 7830 Derwent Publications Ltd., London, GB; Class A41, AN 78-54215A XP002081240 & JP 53 068762 A (AGENCY OF IND SCI & TECHNOLOGY), 19. Juni 1978

## Beschreibung

Die Erfindung betrifft ein verbessertes Verfahren zur Herstellung und Reinigung von 3-(4-Hydroxyphenyl)-1,1,3-trimethylindan-5-ol durch Isomerisierung von Dimeren oder Oligomeren des Isopropenylphenols und anschließende Umkristallisation.

DE-A-2 645 020 offenbart die Umkristallisation von Hydroxytrimethylhydroxyphenylindan aus einem Essig/Wasser-Gemisch.

Für die Herstellung von 3-(4-Hydroxyphenyl)-1,1,3-trimethylindan-5-ol, im folgenden Bisphenol-Indan genannt, sind bereits verschiedene Verfahren bekannt geworden.

So offenbaren US-A 2 754 285 und US-A 2 819 249 einen Herstellungsweg über die säurekatalysierte Dimerisierung von α-Methylstyrol zu Indanen, welche anschliessend sulfoniert und dann mit Kaliumhydroxid verseift werden.

US-A 2 979 534 lehrt, daß die durch Spaltung von Bisphenolen erhaltenen monomeren Isopropenylphenole in Gegenwart aromatischer Sulfonsäuren oder Mineralsäuren bei Temperaturen von 110 bis 160°C zu Bisphenol-Indan dimerisiert werden können. Bisphenolspaltung und Indanbildung können auch in einem Schritt durchgeführt werden. Nach diesem Verfahren wurde ein Produkt geringer Reinheit erhalten, das auch nach Rekristallisation aus Benzol/Cyclohexan nur einen Schmelzpunkt von 165-166°C aufwies.

US-A 3 264 357 offenbart die Herstellung von Bisphenolen durch Reaktion einer Mischung der beiden isomeren Formen des dimeren Isopropenylphenols mit Phenolen in Gegenwart starker Säuren. Es wird berichtet, daß in Abwesenheit reaktiver Phenole bei einer Reaktionstemperatur von 90°C Bisphenol-Indan gebildet wird. Nach US-A 3 264 358 kann Bisphenol-Indan durch Umsetzung einer Mischung der beiden isomeren Formen des dimeren Isopropenylphenols mit stark sauren Katalysatoren erhalten werden, beispielsweise durch zweistündiges Tempern in konzentrierter Salzsäure bei Siedehitze.

US-A 3 288 864 offenbart die Herstellung von Bisphenol-Indan durch Selbstkondensation von monomerem Isopropenylphenol in Gegenwart von Friedel-Crafts-Katalysatoren bei Temperaturen von 50 bis 150°C, JP-A 60/35150 lehrt die Isomerisierung von Isopropenylphenol oder dessen Oligomeren in Gegenwart fester Katalysatoren wie Aluminiumoxid oder Terra alba.

Nach US-A 4 334 106 kann Bisphenol-Indan durch Umsetzung von Isopropenylphenol oder dessen Oligomeren in Halogencarbonsäuren oder Ameisensäure bei Temperaturen von 0 bis 90°C hergestellt werden.

Gemäß JP-A 5/294879 kann Bisphenol-Indan durch thermische Zersetzung von Bisphenol-A in Gegewart von aktiviertem Ton erhalten werden, US-A 3 271 463 offenbart die Bildung von Bisphenol-Indan als Nebenprodukt bei der Behandlung von Bisphenol A mit wäßriger Schwefelsäure bei 90-150°C. Bei beiden Verfahren werden größere Mengen an Spirobisindan-Bisphenol gebildet, welches durch Umkristallisation aus aromatischen Kohlenwasserstoffen abgetrennt werden muß.

Die beschriebenen Verfahren sind für eine industrielle Produktion von Bisphenol-Indan zur Verwendung als Ausgangsstoff für die Kunststoffherstellung vielfach noch unzureichend, was die Ausbeuten und die Reinheit des Produkts angeht. Es wurde nun ein verbessertes Verfahren gefunden, durch das sich Bisphenol-Indan in hoher Ausbeute und Reinheit herstellen läßt.

Gegenstand der Erfindung ist ein Verfahren zur Herstellung und Reinigung von Bisphenol-Indan, bei dem man zunächst Isopropenylphenol, dessen Dimere oder Oligomere oder deren Mischungen in einem organischen Lösungsmittel löst, dessen Siedepunkt im Temperaturbereich von 110 bis 150°C, bevorzugt 130 bis 140°C liegt, das Reaktionsgemisch auf eine Temperatur im Bereich von 60 bis 110°C, bevorzugt 70 bis 90°C erwärmt, anschließend 0,002 bis 5 Gew.-%, bevorzugt 0,3 bis 0,5 Gew.-%, bezogen auf die eingesetzte Menge an Isopropenylphenol, eines sauren Katalysators gegebenenfalls portionsweise zusetzt, die Reaktionsmischung zum Sieden erhitzt und bei Siedetemperatur 1 bis 600 Minuten lang, bevorzugt 2 bis 60 Minuten lang reagieren läßt, dann gegebenenfalls die Reaktionsmischung bei einer Temperatur im Bereich von 60 bis 100°C, bevorzugt 70 bis 90°C, durch Zugabe einer Base neutralisiert, anschließend der Reaktionsmischung Wasser zusetzt und sie auf eine Temperatur im Bereich von 0 bis 30°C, bevorzugt 0 bis 10°C, abkühlt, den gebildeten Niederschlag abtrennt, trocknet und aus einem Essigsäure/Wasser-Gemisch umkristallisiert.

Als Ausgangsstoffe für das erfindungsgemäße Verfahren werden Isopropenylphenol, dessen Dimere oder Oligomere eingesetzt. Die Dimere und Oligomere sind leicht zugänglich und können beispielsweise nach den in US-A 3 288 864 oder USA 4 201 877 beschriebenen Methoden hergestellt werden.

Im erfindungsgemäßen Verfahren werden als Lösungsmittel solche organischen Lösungsmittel verwendet, die einen Siedepunkt im Temperaturbereich von 110 bis 150°C, bevorzugt 130 bis 140°C aufweisen. Beispiele sind Toluol, n-Butanol, Chlorbenzol und Xylol, bevorzugt wird Chlorbenzol verwendet. Die Menge an Lösungsmittel beträgt bevorzugt das zwei- bis dreifache der eingesetzten Menge an Isopropenylphenol.

Nach Erwärmen auf eine Temperatur im Bereich von 60 bis 110°C, bevorzugt 70 bis 90°C, werden der Reaktionsmischung 0,002 bis 5 Gew.-%, bevorzugt 0,3 bis 0,5 Gew.-%, bezogen auf die eingesetzte Menge an Isopropenylphenol, eines sauren Katalysators zugesetzt. Als Katalysatoren für das erfindungsgemäße Verfahren können Brönstedt- oder Lewis-Säuren verwendet werden. Beispiele sind Mineralsäuren wie Salzsäure oder Schwefelsäure, organische Säuren wie Sulfonsäuren oder Halogencarbonsäuren, saure Ionentauscherharze, Bortrifluorid und Metallhalogenide wie AlCl₃, FeCl₃ oder ZnCl₂. Bevorzugt werden als Katalysatoren Lewis-Säuren eingesetzt, besonders bevorzugt Bortrifluorid.

Nach Zugabe des Katalysators erhitzt man die Reaktionsmischung zum Sieden und läßt bei Siedetemperatur 1 bis 600 Minuten lang, bevorzugt 2 bis 60 Minuten lang reagieren. Es hat sich gezeigt, daß die Isomerisierungsreaktion bei Temperaturen im Bereich von 110 bis 150°C, insbesondere 130 bis 140°C, mit besonders hoher Selektivität zu Bisphenol-Indan führt.

Gegebenenfalls wird anschließend die Reaktionsmischung bei einer Temperatur im Bereich von 60 bis 100°C, bevorzugt 70 bis 90°C, durch Zusatz einer Base neutralisiert. Hierzu ist eine Vielzahl verschiedener Basen oder deren Mischungen geeignet. Beispiele sind Metallhydroxide wie NaOH, KOH, Mg(OH)₂, Ca(OH)₂, Alkoholate wie Natriummethanolat, Natriumethanolat, Natriumphenolat, Kaliummethanolat, Kaliumethanolat, Kaliumphenolat, Magnesiummethanolat, Magnesiumethanolat, Magnesiumphenolat, Calciummethanolat, Calciumethanolat, Calciumphenolat, Aluminiumisopropylat, Carboxylate wie Natriumformiat, Natriumacetat, Natriumbenzoat, Calciumformiat, Calciumacetat, Carbonate wie Natriumcarbonat, Kaliumcarbonat, Calciumcarbonat, Magnesiumcarbonat, (NH₄)₂CO₃, Hydrogencarbonate wie NaHCO₃, KHCO₃ oder NH₄HCO₃, Mischungen aus NH₄HCO₃ und Ammoniumcarbamat, Ammoniak, Amine wie Triethylamin, Diethylamin, Ethylamin, Trimethylamin, Dimethylamin, Methylamin und deren Lösungen in Wasser oder organischen Lösungsmitteln, die nicht mit dem Reaktionsmedium mischbar sind. Bevorzugt wird als Base wäßrige Natronlauge verwendet. Wird eine Base verwendet, die mit dem Reaktionsgemisch ein Zweiphasensystem bildet, so wird nach der Neutralisation die das Bisphenol-Indan enthaltende organische Phase abgetrennt, bevor ihr weiteres Wasser zugesetzt wird.

Die Reaktionsmischung wird mit Wasser versetzt und abgekühlt, um das Bisphenol-Indan zu isolieren. Die zugesetzte Wassermenge beträgt bevorzugt ein Viertel bis ein Drittel der Menge an Reaktionsmischung. Man kühlt auf eine Temperatur im Bereich von 0 bis 30°C, bevorzugt 0 bis 10°C, ab. Bevorzugt wird die Reaktionsmischung für 1 bis 200 Minuten, besonders bevorzugt 40 bis 80 Minuten bei dieser Temperatur gehalten, bevor man den gebildeten Niederschlag abtrennt. Dies kann durch die dem Fachmann bekannten Methoden geschehen, z.B. durch Filtrieren, Dekantieren oder Zentrifugieren. Vorzugsweise wird der Niederschlag anschließend mit einem organischen Lösungsmittel, z.B. Chlorbenzol, gewaschen. Es zeigt sich, daß ein deutlich weniger gefärbtes Produkt erhalten wird, wenn die Reaktionsmischung vor der Wasserzugabe neutralisiert wurde.

In einer bevorzugten Ausführungsform des Verfahrens wird der Niederschlag nach der Abtrennung aus der Reaktionsmischung für 5 bis 100 Minuten bei Temperaturen im Bereich von 10 bis 50°C, bevorzugt 20 bis 30°C, in Wasser suspendiert. Die verwendete Wassermenge beträgt dabei bevorzugt das ein- bis zehnfache der Menge an Niederschlag. Anschließend wird der Niederschlag erneut abgetrennt. In einer bevorzugten Ausführungsform wird er dann nochmals bei 10 bis 50°C mit der ein- bis zehnfachen Menge an Wasser gespült. Der erhaltene Feststoff wird getrocknet, bevorzugt bei Temperaturen von 20 bis 150°C, besonders bevorzugt bei 70 bis 90°C. Die Trocknung erfolgt bevorzugt unter vermindertem Druck. Anschließend wird das so erhaltene Rohprodukt aus einem Essigsäure/Wasser-Gemisch umkristallisiert.

Bei der Reinigung des rohen Bisphenol-Indans durch Umkristallisation wurden bislang Gemische von Benzol und Cyclohexan oder Methanol und Wasser eingesetzt. Die Reinheit der so erhaltenen Produkte ist für den Einsatz in der industriellen Kunststoffproduktion noch unbefriedigend. Es wurde nun gefunden, daß sich Bisphenol-Indan höherer Reinheit erhalten läßt, wenn das Rohprodukt aus Gemischen von Essigsäure und Wasser umkristallisiert wird.

Gegenstand der Erfindung ist daher auch ein Verfahren zur Reinigung von Bisphenol-Indan durch Umkristallisation aus Essigsäure/Wasser-Gemischen. Bevorzugt enthalten diese Gemische Essigsäure und Wasser im Verhältnis 1 zu 2 bis 3 zu 2, besonders bevorzugt enthalten sie 57 bis 63 % Essigsäure.

Zur Umkristallisation kann das Rohprodukt in Wasser vorgelegt werden. Nach Erwärmen auf 70 bis 100 °C wird Essigsäure zugegeben, bis der Feststoff gelöst ist. Alternativ kann das Rohprodukt auch bei 70 bis 100°C direkt in einem Essigsäure/Wasser-Gemisch gelöst werden. Durch darauffolgendes Abkühlen wird das Produkt ausgefällt. Es ist auch möglich, das Rohprodukt zunächst in Essigsäure bei 70 bis 100 °C zu lösen und Bisphenol-Indan dann mit Wasser zu fällen. Das Produkt wird abgetrennt, mit Wasser gewaschen und getrocknet, bevorzugt unter vermindertem Druck bei Temperaturen von 20 bis 150 °C.

Die Kristallisation kann nach den dem Fachmann bekannten Methoden durchgeführt werden, z.B. durch Mehrfachkristallisation, stufenweise Kristallisation oder Fällungskristallisation.

Aufgrund seiner hohen Reinheit ist das nach dem erfindungsgemäßen Verfahren hergestellte Bisphenol-Indan in hervorragender Weise geeignet als Ausgangsstoff für die Herstellung von hochwertigen Kunststoffen, z.B. Polycarbonaten.

### Beispiele

### Beispiel 1a

40 g dimeres Isopropenylphenol wurden in 100 ml Chlorbenzol gelöst. Nach Erwärmen der Reaktionsmischung auf 80°C wurden 0.14 ml BF₃ als Etherat zugegeben. Die Reaktionslösung wurde zum Sieden erhitzt und dann für 40 Minuten bei 132°C unter Rückfluß gehalten. Die Reaktionsmischung wurde auf 0 bis 10°C abgekühlt und mit 30 ml Wasser versetzt. Nach 60 Minuten wurde der Niederschlag abgetrennt und mit 50 ml Chlorbenzol portionsweise gewaschen. Es wurden 36 g Produkt (entsprechend 90 % der Einwaage an Isopropenylphenol) mit einem Bisphenol-Indan-Gehalt von 90 % erhalten.

Der Rückstand wurde in 100 ml Wasser suspendiert, nach 15 Minuten wieder isoliert und nochmals portionsweise mit 100 ml Wasser versetzt. Das Produkt wurde abgetrennt und am Wasserstrahlvakuum bei 80°C getrocknet. Das getrocknete Produkt wurde aus Essigsäure/Wasser umkristallisiert, indem es zunächst bei 100°C in Wasser suspendiert und der Suspension dann soviel Essigsäure zugegeben wurde, daß sich der Bodensatz löste. Beim darauffolgenden Abkühlen kristallisierte das Produkt aus. Der Feststoff wurde gesammelt, kurz mit Wasser gewaschen und unter vermindertem Druck bei 80°C getrocknet. Es wurden 24 g (entsprechend 60 % der Einwaage an Isopropenylphenol) Produkt mit einem Bisphenol-Indan-Gehalt von 97,7 % erhalten.

### Beispiel 1b

800 g dimeres Isopropenylphenol wurden in 2000 ml Chlorbenzol gelöst. Nach Erwärmen der Reaktionsmischung auf 80°C wurden 2,8 ml BF₃ als Etherat zugegeben. Die Reaktionslösung wurde zum Sieden erhitzt und dann für 40 Minuten bei 132°C unter Rückfluß gehalten. Anschließend wurde der Katalysator in der Reaktionslösung durch Zugabe von Natronlauge (1.164 g NaOH auf 300 ml Wasser) bei 80°C neutralisiert. Die wäßrige Phase wurde abgetrennt, die organische Phase auf 0 bis 10°C abgekühlt und mit 600 ml Wasser versetzt. Nach 60 Minuten wurde der Niederschlag abgetrennt und mit 1000 ml Chlorbenzol portionsweise gewaschen. Es wurden 480 g Produkt (entsprechend 60 % der Einwaage an Isopropenylphenol) mit einem Bisphenol-Indan-Gehalt von 89 % erhalten.

Der Rückstand wurde in 1000 ml Wasser suspendiert, nach 15 Minuten wieder isoliert und nochmals portionsweise mit 1000 ml Wasser versetzt. Das Produkt wurde abgetrennt und am Wasserstrahlvakuum bei 80°C getrocknet. Das getrocknete Produkt wurde aus Essigsäure/Wasser umkristallisiert, indem es man bei 100°C in einem Essigsäure/Wasser-Gemisch löste. Beim darauffolgenden Abkühlen kristallisierte das Produkt aus. Der Feststoff wurde gesammelt, kurz mit Wasser gewaschen und unter vermindertem Druck bei 80°C getrocknet. Es wurden 312 g (entsprechend 65 % der Einwaage an Rohprodukt) Produkt mit einem Bisphenol-Indan-Gehalt von 97,2 % erhalten.

### Beispiel 2 (Vergleichsbeispiel analog US-A 3 288 864)

40 g dimeres Isopropenylphenol wurden in 100 ml Toluol gelöst. Nach Erwärmen der Reaktionsmischung auf 80°C wurden 0,14ml BF₃ als Etherat zugegeben. Die Reaktionslösung wurde dann 40 Minuten lang bei 85°C getempert, anschließend auf 0 bis 10°C abgekühlt und mit 30 ml Wasser versetzt. Nach 60 Minuten wurde der Niederschlag abgetrennt und mit 50 ml Toluol portionsweise gewaschen. Der Rückstand wurde in 100 ml Wasser suspendiert, nach 15 Minuten wieder isoliert und nochmals portionsweise mit 2 l Wasser versetzt. Nach erneuter Abtrennung und Trocknung unter vermindertem Druck bei 80°C wurden 36 g Produkt (entsprechend 90 % der Einwaage an Isopropenylphenol) mit einem Bisphenol-Indan-Gehalt von 83 % erhalten. Nach Umkristallisation aus Methanol/Wasser wurdne 18 g (entsprechend 45 % der Einwaage an Isopropenylphenol) Produkt mit einem Bisphenol-Indan-Gehalt von 92 % erhalten.

### Beispiel 3a

50 g rohes Bisphenol-Indan (Smp. 186°C, Bisphenol-Indan-Gehalt 86,4 %) wurden in 200 ml Wasser suspendiert. Bei Siedehitze wurden 150 ml Essigsäure zugegeben. Aus der erhaltenen klaren Lösung konnten nach Abkühlen 34 g Produkt (68 %) mit einem Bisphenol-Indan-Gehalt von 93,2 % isoliert werden.

### Beispiel 3b

50 g des rohen Bisphenol-Indans wurden bei Siedehitze in einem Gemisch von 79,8 ml Wasser und 60,2 ml Essigsäure gelöst. Nach Abkühlen wurden 31,5 g Produkt (63 %) mit einem Bisphenol-Indan-Gehalt von 93,5 % erhalten.

### Beispiel 3c (Vergleich)

50 g des rohen Bisphenol-Indans wurden 100 ml Wasser suspendiert. Unter Rückfluß 95 ml Methanol zugegeben. Aus der klaren Lösung kannten nach Abkühlen 38,4 g Produkt (76,8 %) mit einem Bisphenol-Indan-Gehalt von 88,8 % isoliert werden.

## Patentansprüche

1. Verfahren zur Herstellung und Reinigung von 3-(4-Hydroxyphenyl)-1,1,3-trimethylindan-5-ol, bei dem man
a) Isopropenylphenol, dessen Dimere oder -oligomere oder deren Mischungen in einem organischen Lösungsmittel löst, dessen Siedepunkt im Temperaturbereich von 110 bis 150°C liegt,
b) das Reaktionsgemisch auf eine Temperatur im Bereich von 60 bis 110°C erwärmt,
c) 0,002 bis 5 Gew.-%, bezogen auf die Menge an Isopropenylphenol, eines sauren Katalysators zusetzt,
d) die Reaktionsmischung zum Sieden erhitzt und bei Siedetemperatur 1 bis 600 Minuten lang reagieren läßt,
e) die Reaktionsmischung gegebenenfalls bei einer Temperatur im Bereich von 60 bis 100°C durch Zugabe einer Base neutralisiert,
f) der Reaktionsmischung Wasser zusetzt und sie auf eine Temperatur im Bereich von 0 bis 30°C abkühlt,
g) den gebildeten Niederschlag abtrennt, trocknet und aus einem Essigsäure/Wasser-Gemisch umkristallisiert.

2. Verfahren gemäß Anspruch 1, bei dem der Niederschlag nach der Abtrennung aus der Reaktionsmischung für 5 bis 100 Minuten bei Temperaturen im Bereich von 10 bis 50°C in Wasser suspendiert, anschließend getrocknet und umkristallisiert wird.

## Claims

1. Process for the preparation and purification of 3-(4-hydroxyphenyl)-1,1,3-trimethylindan-5-ol, wherein
a) isopropenylphenol, its dimers or oligomers or mixtures of these are dissolved in an organic solvent, the boiling point of which is in the temperature range of 110°C to 150°C,
b) the reaction mixture is heated to a temperature within the range of 60°C to 110°C,
c) 0.002 to 5 wt.%, based on the quantity of isopropenylphenol used, of an acidic catalyst is added,
d) the reaction mixture is heated to boiling and allowed to react at the boiling temperature for 1 to 600 minutes,
e) the reaction mixture is optionally neutralised at a temperature within the range of 60°C to 100°C by addition of a base,
f) water is added to the reaction mixture and the latter is cooled to a temperature within the range of 0°C to 30°C,
g) the precipitate formed is separated off, dried and recrystallised from an acetic acid/water mixture.

2. Process according to claim 1, wherein the precipitate, after having been separated from the reaction mixture, is suspended in water for 5 to 100 minutes at temperatures within the range of 10°C to 50°C, then dried and recrystallised.

## Revendications

1. Procédé de préparation et d'épuration de 3-(4-hydroxyphényl)-1,1,3-triméthylindan-5-ol, dans lequel :
a) on dissout l'isopropénylphénol, ses dimères ou oligomères ou leurs mélanges, dans un solvant organique, dont la température d'ébullition se situe dans l'intervalle allant de 110 à 150°C ;
b) on chauffe le mélange réactionnel à une température située dans l'intervalle allant de 60 à 110°C ;
c) on ajoute 0,002 à 5% en poids sur base de la quantité d'isopropénylphénol, d'un catalyseur acide ;
d) on chauffe le mélange réactionnel jusqu'à ébullition et on laisse réagir à la température d'ébullition pendant 1 à 600 minutes ;
e) on neutralise le mélange réactionnel, le cas échéant à une température située dans l'intervalle allant de 60 à 100°C, par addition d'une base ;
f) on ajoute de l'eau au mélange réactionnel et on refroidit jusqu'à une température située dans l'intervalle allant de 0 à 30°C, et
g) on sépare le résidu formé, on sèche et on recristallise à partir d'un mélange acide acétique/eau.

2. Procédé suivant la revendication 1, dans lequel après la séparation du mélange réactionnel, le résidu est mis en suspension dans de l'eau pendant 5 à 100 minutes à des températures situées dans l'intervalle allant de 10 à 50°C, puis on sèche et on recristallise.
